# EUROPÄISCHE PATENTSCHRIFT

(11) **EP 2 788 712 B1**
(45) Veröffentlichungstag und Bekanntmachung des Hinweises auf die Patenterteilung: **15.11.2017**
(21) Anmeldenummer: 12808727.7
(22) Anmeldetag: 07.12.2012
(51) Int. Cl.: B65H 49/02, A61B 5/11, G01B 5/004

(54) **REHABILITATIONSGERÄT MIT EINEM POSITIONSSENSOR, VERWENDUNG DES POSITIONSSENSORS IN EINEM REHABILITATIONSGERÄT**
REHABILITATION DEVICE WITH A POSITION SENSOR, USE OF THE POSITION SENSOR IN A REHABILITATION DEVICE
APPAREIL DE RÉÉDUCATION AVEC CAPTEUR DE POSITION, UTIISATION DU CAPTEUR DE POSITION DANS UN APPAREIL DE RÉÉDUCATION

(30) Priorität: 09.12.2011 DE 102011056219
(43) Veröffentlichungstag der Anmeldung: 15.10.2014
(73) Patentinhaber: Tyromotion GmbH, 8020 Graz (AT)
(72) Erfinder: RAM, David, A-8045 Graz (AT); KOLLREIDER, Alexander, A-8111 Judendorf-Straßengel (AT); PITSCH, Harald, A-8045 Graz (AT); CEE, Valentin, A-8063 Eggersdorf (AT); NEUBAUER, Josef, A-8230 Hartberg (AT)
(74) Vertreter: Wirnsberger, Gernot
(86) Internationale Anmeldenummer: PCT/EP2012/074813
(87) Internationale Veröffentlichungsnummer: WO 2013/083788

(56) Entgegenhaltungen:
- WO-A1-2010/147574
- US-B1- 6 785 973

## Beschreibung

Die Erfindung betrifft ein Rehabilitationsgerät mit einem Positionssensor, sowie die Verwendung eines Positionssensors für ein Rehabilitationsgerät. Rehabilitationsgeräte, insbesondere für Patienten mit verminderter Funktionsfähigkeit der oberen Extremitäten, weisen in einigen Ausführungsformen einen Positionssensor zur Bestimmung einer Lage der Extremität des Patienten auf. Dazu umfasst ein solcher Positionssensor beispielsweise ein Seilsystem, bei dem an einem Ende des Teils eine Schlaufe oder eine Schlinge für die Aufnahme der Extremität, beispielsweise eines Arms, des Patienten vorgesehen ist. Das Seil wird beispielsweise über eine Rolle von einem Gewichtssystem geführt, wobei das Gewichtssystem eine Entlastung des Arms des Patienten bewirkt. Um eine Position des Arms festzustellen, wird beispielsweise eine bestimmte Seillänge im Seilsystem ermittelt, und zusätzlich eine Kamera verwendet, um auf der Basis des Kamerasignals und der ermittelten Seillänge die Position zu berechnen. WO 2010/147574 A1 und US 6 785 973 B1 offenbaren einen Positionssensor. Eine zu lösende Aufgabe besteht darin, ein verbessertes Konzept zur Positionsbestimmung anzugeben, welche sich insbesondere in einem Therapiegerät einsetzen lässt.

Diese Aufgabe wird mit dem Gegenstand der unabhängigen Patentansprüche gelöst. Weiterbildungen und Ausgestaltungsformen sind Gegenstand der abhängigen Ansprüche.

Der vorgeschlagenen Lösung liegt die Idee zugrunde, dass ein Seil über eine Spuleinrichtung mit einem elektrischen Antrieb abgespult und aufgespult werden kann, wobei durch das Aufspulen und Abspulen gleichzeitig eine Abspullänge des abgespulten Seils ermittelt wird. Das Seil wird über eine Führungseinrichtung von der Spuleinrichtung weggeführt, wobei die Führungseinrichtung eine feste Zentriereinrichtung und eine kardanisch gelagerte Auslassvorrichtung aufweist. Über einen Lagesensor an der beweglichen Auslassvorrichtung kann somit ein Auslasswinkel des Seils bestimmt werden. Der Positionssensor ist hierbei eingerichtet, aus der ermittelten Abspullänge und dem Austrittswinkel des Seils die Lage eines vorbestimmten Punkts des Seils, beispielsweise des Seilendes, an dem eine Schlaufe befestigt ist, zu bestimmen. Dementsprechend kann mit der vorgeschlagenen Lösung eine Positionsbestimmung mit hoher Genauigkeit und vergleichsweise geringen räumlichen Bedarf bereitgestellt werden. Durch eine Kraftmesseinrichtung und den elektrischen Antrieb ist es möglich, mit einer gezielt einstellbaren Kraft auf das Seil beziehungsweise ein an dem Seil befestigtes Objekt einzuwirken.

Wenn zwei solcher Positionssensoren kombiniert werden, kann anhand der ermittelten Raumpositionen der jeweiligen Seilpunkte die Lage eines Objekts im Raum, welches an den Seilpunkten befestigt ist, mit geringem Aufwand ermittelt werden. Dadurch eignen sich solche Positionssensoren insbesondere für ein Therapiegerät, bei dem an den Seilenden ein Arm an zwei Schlaufen befestigt beziehungsweise gehalten wird, um beispielsweise die Lage des Arms zu erfassen und grafisch zu visualisieren.

In einer Ausführungsform umfasst ein Positionssensor eine Halterung, eine fest mit der Halterung verbundene Kraftmesseinrichtung und eine fest mit der Kraftmesseinrichtung verbundene Spuleinrichtung mit einem Antrieb, der einen Elektromotor aufweist. Die Spuleinrichtung ist eingerichtet zum Abspulen und Aufspulen eines Seils und zur Ermittlung einer Abspullänge des abgespulten Seils. Der Positionssensor umfasst ferner eine fest mit der Halterung verbundene Führungseinrichtung, die eine Zentriereinrichtung zur definierten, insbesondere richtungsfesten Durchführung des Seils, eine kardanisch gelagerte Auslassvorrichtung zur richtungsvariablen Durchführung des Seils und einen Lagesensor zur Ermittlung einer Winkellage der Auslassvorrichtung aufweist. Der Positionssensor ist eingerichtet, die Lage eines vorbestimmten Punkts des Seils relativ zur Halterung auf der Basis der ermittelten Winkellage und der Abspullänge zu bestimmen. Der Elektromotor ist eingerichtet, mit einer einstellbaren Zugkraft auf das Seil einzuwirken, die auf der Basis einer Kraft eingestellt wird, welche über das Seil auf die Halterung wirkt und welche von der Kraftmesseinrichtung ermittelt wird.

Die Führungseinrichtung ermöglicht eine präzise Bestimmung der Winkellage des Seils im Raum. Insbesondere lässt sich über die Führungseinrichtung aufgrund der zwei definierten Durchführungen des Seils, nämlich in der Zentriereinrichtung und der Auslassvorrichtung, ein Ortsvektor beziehungsweise Richtungsvektor für die Richtung des Seils außerhalb des Positionssensors ermitteln. Hierbei ist die Durchführung in der Zentriereinrichtung ein fest vorgegebener Punkt, während die Durchführung durch die Auslassvorrichtung wegen der kardanischen Lagerung einen variablen Punkt liefert. Aus diesen zwei Punkten kann mit bekannten Verfahren der Vektorgeometrie der Ortsvektor bestimmt werden. Die Genauigkeit der Bestimmung ergibt sich daraus, dass über den Lagesensor eine exakte Orientierung der Auslassvorrichtung bestimmt werden kann. Beispielsweise umfasst der Lagesensor einen oder mehrere Beschleunigungssensoren, welche eine Winkellage der Auslassvorrichtung und damit eine Bestimmung des Punktes der Durchführung für das Seil in der Auslassvorrichtung ermöglichen.

Basierend auf dem ermittelten Ortsvektor und der Abspullänge des Seils kann somit die Lage des vorbestimmten Punkts des Seils im Raum beziehungsweise relativ zur Halterung beziehungsweise dem Positionssensor ermittelt werden. Hierfür ist es lediglich nötig, die Entfernung des vorbestimmten Punkts zum Positionssensor in einem Referenzzustand mit einer bestimmten Abspullänge zu kennen. Diese lässt sich beispielsweise durch Programmierung oder Konfiguration des Positionssensors einstellen. Beispielsweise ist für die Bestimmung der Abspullänge an der Spuleinrichtung ein Encoder vorgesehen, welcher die Zahl der Umdrehungen einer Spule zu zählen, wobei auch teilweise Umdrehungen mit einer vorbestimmten Genauigkeit eingerechnet werden. Aus dieser Umdrehungszahl, die auch nicht ganzzahlig sein kann, kann über den Radius der Spule beziehungsweise einen Aufwickelradius die Abspullänge ermittelt werden. Um die Bestimmung der Abspullänge möglichst genau ermitteln zu können, ist die Spuleinrichtung vorzugsweise derart ausgestaltet, dass das Seil einlagig nebeneinander, also mit gleichbleibendem Wicklungsradius, oder einlagig übereinander, also mit kontinuierlich steigendem Wicklungsradius, aufgewickelt wird. Jedoch kann bei dünneren Seilstärken auch bei beliebiger Aufwicklung eine ausreichend hohe Genauigkeit für die Ermittlung der Abspullänge erreicht werden, beispielsweise durch Annahme eines mittleren Wicklungsradius.

In verschiedenen Ausführungsformen weist der Antrieb auch eine Aufspulfeder auf, welche beispielsweise zu einer automatischen Aufwicklung des Seils mit einer vorgegebenen Kraft beiträgt. Alternativ oder zusätzlich kann der Antrieb auch einen Elektromotor aufweisen, welcher die Spule der Spuleinrichtung antreibt. Der Elektromotor ist beispielsweise eingerichtet, mit einer einstellbaren Zugkraft auf das Seil einzuwirken. Dementsprechend wird eine an dem Seil befestigte Last mit dieser Kraft nach oben gezogen. Dadurch ergibt sich beispielsweise, dass wenn die Last eine größere Kraft als die eingestellte Kraft des Motors nach unten bewirkt, sich das Seil abspult. Andererseits wird das Seil aufgespult, wenn die am Seil befestigte Last mit einer geringeren Kraft als der vom Motor eingestellten Kraft wirkt.

Beispielsweise wird die Zugkraft des Elektromotors auf der Basis einer Kraft eingestellt, welche über das Seil auf die Halterung wirkt und welche von der Kraftmesseinrichtung ermittelt wird. Damit kann beispielsweise eine am Seil befestigte Last in einer Ruheposition gehalten werden. Weiterhin ist es möglich, beispielsweise in einer Kalibrierungsphase, die auf das Seil wirkende Kraft der Last zu bestimmen, um diese dann der Einstellung der Zugkraft des Motors zugrunde zu legen. Somit führen Laständerungen in positiver und negativer Richtung am Seil zu einem Abspulen beziehungsweise Aufspulen des Seils durch die Spuleinrichtung.

Beim Einsatz eines Elektromotors für den Antrieb kann die Abspullänge beispielsweise über einen mit dem Elektromotor verbundenen Motorencoder bestimmt werden. Dazu weist der Motor in einigen Ausführungsformen Positionssensoren oder Drehsensoren auf, welche eine Lage des Motors bestimmen. Alternativ oder zusätzlich können auch elektrische Parameter beim Betrieb des Motors ausgewertet werden, um einen Drehwinkel des Motors zu überwachen.

Durch die kardanische Lagerung der Auslassvorrichtung ist es möglich, dass sich diese zumindest entlang von zwei Raumachsen bewegen beziehungsweise drehen lässt. Insbesondere lässt sich die Winkellage der Auslassvorrichtung über zwei Raumwinkel darstellen, einen Azimutwinkel und einen Elevationswinkel, wobei der Azimutwinkel einen Winkelbereich von etwa 0° bis etwa 360° umfasst und der Elevationswinkel einen Winkel von etwa 0° bis etwa 180°, beziehungsweise von etwa -90° bis etwa +90° umfasst. Aus diesen Winkeln und dem bekannten Punkt der richtungsvariablen Durchführung für das Seil kann somit der variable Durchführungspunkt zur Bestimmung des Ortsvektors beziehungsweise Richtungsvektors des Seils berechnet werden. Wenn der Punkt der Durchführung in der Zentriereinrichtung mit dem Drehzentrum der Auslassvorrichtung zusammenfällt, entspricht der Richtungsvektor des Seils beispielsweise unmittelbar der Winkellage der Auslassvorrichtung.

Ein Positionssensor in einer der beschriebenen Ausführungsformen kann beispielsweise für ein Rehabilitationsgerät oder Therapiegerät verwendet werden, wobei an dem vorbestimmten Punkt des Seils eine Schlaufe oder eine Schlinge vorgesehen wird, durch die eine Extremität, insbesondere ein Arm eines Patienten, gehalten wird. Durch die einstellbare Kraft beziehungsweise durch die Kraftmessung kann somit die Extremität des Patienten entlastet werden, sodass der Patient weniger eigene Kraft benötigt, um die Extremität, insbesondere den Arm, zu halten. Die Bestimmung der Position des vorbestimmten Punktes beziehungsweise der Schlaufe oder Schlinge ermöglicht es, die Bewegung des Patienten nachzuvollziehen und beispielsweise grafisch zu visualisieren. Dadurch, dass keine externen Komponenten wie beispielsweise eine Kamera an den Positionssensor notwendig sind, um eine exakte Position zu bestimmen, lässt sich ein derartiges Therapiegerät oder Rehabilitationsgerät mit geringem Aufwand herstellen beziehungsweise installieren und betreiben.

In verschiedenen Ausführungsformen können auch mehrere solcher Positionssensoren kombiniert werden. Beispielsweise umfasst eine Ausführungsform einer Sensoranordnung einen ersten und einen zweiten Positionssensor gemäß einer der beschriebenen Ausführungsformen, wobei der erste Positionssensor und der zweite Positionssensor in einer gemeinsamen Halterung angeordnet sind. Insbesondere sind dadurch die jeweiligen Kraftmesseinrichtungen und die Führungseinrichtungen der beiden Positionssensoren mit einer gemeinsamen Halterung fest verbunden. Beispielsweise ist hierbei die Halterung des ersten Positionssensors gleichzeitig die Halterung des zweiten Positionssensors.

In verschiedenen Ausführungsformen können auch weitere Positionssensoren in der Sensoranordnung integriert werden, welche wiederum auf den zuvor beschriebenen Ausführungsbeispielen basieren.

Mit jedem der Positionssensoren kann dennoch unabhängig voneinander die Position eines jeweils vorbestimmten Punktes am jeweiligen Seil bestimmt werden. Dadurch ist es möglich, auf Basis der ermittelten Punkte im Raum der beiden Seile die Lage eines Objekts relativ zur gemeinsamen Halterung zu bestimmen, welches am vorbestimmten Punkt des Seils des ersten Positionssensors und dem vorbestimmten Punkt des Seils des zweiten Positionssensors befestigt ist. Insbesondere kann beispielsweise wiederum über bekannte vektorgemoetrische Berechnungen aus den zwei bestimmten Raumpunkten ein Ortsvektor für die Lage des Objekts im Raum beziehungsweise relativ zur Halterung berechnet werden.

In weiteren Ausgestaltungsformen ist die Sensoranordnung darauf basierend eingerichtet, die Lage des Objekts grafisch auf einer Anzeigevorrichtung zu visualisieren. Die Sensoranordnung kann dazu beispielsweise eine Recheneinheit und die Anzeigevorrichtung umfassen. Eine solche Recheneinheit kann beispielsweise auch durch einen herkömmlichen Computer gegeben sein, welcher mit den Positionssensoren verbunden ist.

In verschiedenen Ausführungsformen der Sensoranordnung ist an dem vorbestimmten Punkt des Seils des ersten Positionssensors und dem vorbestimmten Punkt des Seils des zweiten Positionssensors jeweils eine Schlinge oder Schlaufe zur Aufnahme einer menschlichen oder tierischen Extremität, insbesondere eines Arms, vorgesehen.

Beispielsweise kann neben der Visualisierung des Objekts, insbesondere eines Arms, auch eine Darstellung in einer virtuellen Realität erfolgen, bei der, gesteuert durch die Bewegung des Arms oder der Extremität, Aktionen ausgelöst werden beziehungsweise Objekte in der virtuellen Realität manipuliert werden.

Eine Sensoranordnung der beschriebenen Art lässt sich wiederum insbesondere für ein Therapiegerät oder Rehabilitationsgerät verwenden, bei der ein Arm eines Patienten mit zwei Schlingen oder Schlaufen gehalten oder befestigt wird. Solche Sensoranordnungen können auch kombiniert werden, um beispielsweise beide Arme eines Patienten gleichzeitig aufzunehmen. Dementsprechend umfasst eine Ausführungsform eines Rehabilitationsgeräts beispielsweise wenigstens zwei Sensoranordnungen gemäß den zuvor beschriebenen Ausführungsformen.

Mit einem solchen Rehabilitationsgerät oder Therapiegerät wird es Patienten mit verminderter Funktionsfähigkeit der oberen Extremität erleichtert, Bewegungen durchzuführen und wieder zu erlernen. Der Patient wird dabei durch die Schlingen beziehungsweise Schlaufen jeweils an zwei Punkten des Arms, beispielsweise am Handgelenk und am Ellbogen an den Seilen des Therapiegeräts verbunden. Wie zuvor beschrieben, ist es durch die Seile der Positionssensoren möglich, den Arm des Patienten ganz oder teilweise von der Schwerkraft zu entlasten, in dem der Arm mit einer einstellbaren Kraft nach oben gezogen wird. Der Zug nach oben wird von den separaten Spuleinrichtungen, die aktiv angetrieben und/oder durch eine Federbelastung angetrieben sind, bewirkt. Durch die Befestigung der Spuleinrichtungen beziehungsweise der Antriebe an der Halterung beziehungsweise einem Gehäuse des Geräts und die dazwischenliegende Kraftmesseinrichtung ist es möglich, permanent und kontinuierlich die Kraft zu messen, mit der der Patient dem Heben des Arms entgegenwirkt, beziehungsweise mit welcher der Patientenarm nach unten zieht. Dadurch ist es auch möglich, dynamisch auf das Verhalten des Patienten zu reagieren und dem Gerät ein intelligentes Verhalten zuzuführen, beispielsweise durch Variation der Zugkraft der Spuleinrichtung.

Wegen der Gewichtsentlastung und der Kraftmessung bietet ein Therapiegerät gemäß einer der beschriebenen Ausführungsformen die Möglichkeit, jederzeit die exakte Lage des Armes, insbesondere des Unterarmes des Patienten zu rekonstruieren. Diese Information kann, wie oben beschrieben, zur Visualisierung des Armes auf einem Bildschirm verwendet werden.

Durch die Verwendung von Seilen ist es während der Therapie möglich, den gesamten Raum vor dem Patienten zur Therapie zu nutzen, ohne durch das Gerät selbst dabei eingeschränkt zu werden. durch eine Sensoranordnung in einer der beschriebenen Ausführungsformen kann jeweils ein Arm behandelt werden. Durch die Kopplung zweier gleichartiger oder identischer Systeme kann auch ein beidarmiger Betrieb ermöglicht werden. Die Ausgestaltung der verwendeten Positionssensoren gemäß den beschriebenen Ausführungsformen ermöglicht die vollständige Bestimmung der Position des Armes, im Gegensatz zu herkömmlichen Therapiegeräten oder Positionssensoren, bei denen weitere, externe Sensoren wie Kameras notwendig sind. Durch die Auswertung der Kraft, die durch den Patientenarm auf die Positionssensoren wirkt, ist eine Regelung der Gewichtsentlastung für den Patientenarm möglich. Eine Sensoranordnung gemäß den beschriebenen Ausführungsformen kann in verschiedenen Ausgestaltungsformen aufgehängt werden, beispielsweise mit einem eigenen Gestell, einer Montage an einer Decke, einer Montage über einem Krankenbett oder dergleichen.

Die Erfindung wird nachfolgend an mehreren Ausführungsbeispielen anhand von Figuren näher erläutert. Gleiche Bezugszeichen kennzeichnen hierbei Elemente oder Bauteile gleicher Funktion. Soweit sich Schaltungsteile oder Bauelemente in ihrer Funktion entsprechen, wird deren Beschreibung nicht in jeder der folgenden Figuren wiederholt.

Es zeigen:
- Figur 1: ein Ausführungsbeispiel eines Positionssensors,
- Figur 2: ein Ausführungsbeispiel einer Sensoranordnung,
- Figur 3: Detailansichten einer Ausführungsform einer Führungseinrichtung, und
- Figur 4: eine Ausführungsform eines Rehabilitationsgeräts.

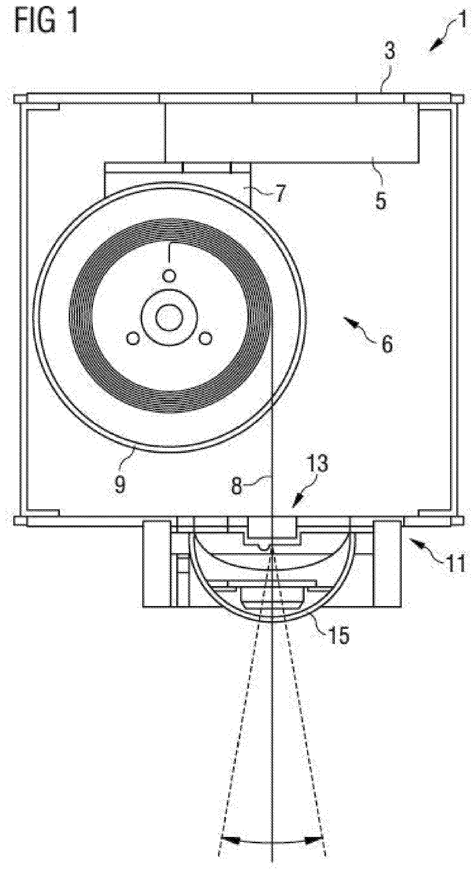

Figur 1 zeigt eine Ausführungsform eines Positionssensors 1 mit einer Halterung 3 beziehungsweise einem Gehäuse. In der Halterung 3 ist eine Spuleinrichtung 6 mit einem Antrieb 7 und einer Spule 9 angeordnet, die über eine Kraftmesseinrichtung 5 mit der Halterung 3 verbunden ist. Über die Kraftmesseinrichtung 5 kann somit die Kraft gemessen werden, welche zwischen der Spuleinrichtung 6 und der Halterung 3 wirkt.

An der Halterung 3 ist ferner eine Führungseinrichtung 11 fest verbunden, welche eine Zentriereinrichtung 13 und eine kardanisch gelagerte Auslassvorrichtung 15 aufweist. Durch die Zentriereinrichtung 13, insbesondere durch den Mittelpunkt der Zentriereinrichtung 13, wird ein auf der Spule 9 aufgewickeltes Seil 8 zentral und ortsfest beziehungsweise richtungsfest durchgeführt. Das Seil 8 wird ferner durch den Mittelpunkt der Auslassvorrichtung 15 geführt, wobei durch die kardanische Lagerung der Auslassvorrichtung 15 eine Orientierung der Auslassvorrichtung veränderbar ist. Beispielsweise wird durch eine Änderung der Zugrichtung auf das Seil 8, dargestellt durch die gestrichelten Linien, im Winkel zur senkrecht verlaufenden Linie des Seils 8, eine Winkellage der Auslassvorrichtung verändert.

Die Führungseinrichtung 11 weist zudem einen hier aus Übersichtsgründen nicht dargestellten Lagesensor auf, der die Ermittlung der Winkellage der Auslassvorrichtung 15 ermöglicht. Der Lagesensor ist vorzugsweise im Inneren der Auslassvorrichtung 15 angeordnet. Aus der ermittelten Winkellage der Auslassvorrichtung 15, welche sich beispielsweise durch einen Azimutwinkel und einen Elevationswinkel darstellen lässt, kann eine Position des Punktes der Durchführung in der Auslassvorrichtung 15 bestimmt werden. Auf Basis dieses Punktes der Durchführung der Auslassvorrichtung 15 und des Punktes der Durchführung des Seils 8 durch die Zentriereinrichtung 13 kann ein Richtungsvektor ermittelt werden, welcher der Austrittsrichtung des Seils 8 aus dem Positionssensor 1 entspricht. Dies kann mit bekannten vektorgeometrischen Verfahren berechnet werden.

Wenn die Auslassvorrichtung kugelförmig ausgestaltet ist, und der Durchführungspunkt durch die Zentriereinrichtung 13 dem Mittelpunkt dieser Kugel entspricht, kann die Winkellage beziehungsweise die Neigung der Auslassvorrichtung 15 unmittelbar als Richtungsvektor des aus dem Positionssensor 1 austretenden Seils 8 verwendet werden.

An der Spuleinrichtung 6 sind entsprechende Sensoren vorgesehen, welche eine Drehung der Spule 9 registrieren, sodass aus einer Anzahl von Umdrehungen der Spule 9 die Länge des abgespulten Seils, ausgehend von einer Anfangslänge, ermittelt werden kann. Bei der vorliegenden Ausführungsform wird das Seil 8 auf der Spule 9 einlagig aufgerollt, sodass das Seil jeweils übereinander und nicht nebeneinander aufgerollt wird. Dementsprechend kann in Abhängigkeit der ermittelten Umdrehungszahl der Spule 9 und dem umdrehungsabhängigen Aufspulradius die Abspullänge des Seils berechnet werden. Alternativ kann das Seil 8 auch einlagig nebeneinander auf einer Spule aufgewickelt werden.

Aus dieser Abspullänge und dem zuvor berechneten Winkel beziehungsweise Ortsvektor des austretenden Seils 8 lässt sich eine Position eines vorbestimmten Punkts des Seils 8 im Raum beziehungsweise relativ zur Halterung 3 berechnen. Dazu weist der Positionssensor 1 beispielsweise eine nicht dargestellte Auswerteeinrichtung mit entsprechender Elektronik auf, welche die notwendigen Berechnungen durchführt. Hierfür kann beispielsweise ein digitaler Signalprozessor oder ein applikationsspezifischer integrierter Schaltkreis, ASIC, eingesetzt werden.

Über die Kraftmesseinrichtung 5 kann insbesondere die Kraft, welche über das Seil 8 auf die Halterung 3 wirkt, bestimmt werden. Ferner kann über den Antrieb 7, beispielsweise einen Elektromotor, eine einstellbare Zugkraft auf das Seil 8 gesteuert werden. Insbesondere lässt sich diese Zugkraft auf der Basis der über die Kraftmesseinrichtung ermittelten Kraft auf das Seil 8 einstellen. Die Zugkraft kann im Betrieb des Positionssensors 1 variiert werden, oder aber nach einer Kalibrierungsphase, welche auch wiederholt werden kann, fest eingestellt werden. Beispielsweise kann an dem Seil eine Schlinge oder Schlaufe befestigt werden, durch die der Arm eines Patienten gehalten wird. In der Kalibrierungsphase kann somit beispielsweise die Kraft des Armes in einer Ruhelage auf die Halterung 3 ermittelt werden, um diese ermittelte Kraft dann als Zugkraft des Antriebs 7 einzustellen. Dementsprechend wird der Arm des Patienten um diese eingestellte Zugkraft entlastet, sodass Bewegungen des Armes mit geringerem Kraftaufwand erfolgen können.

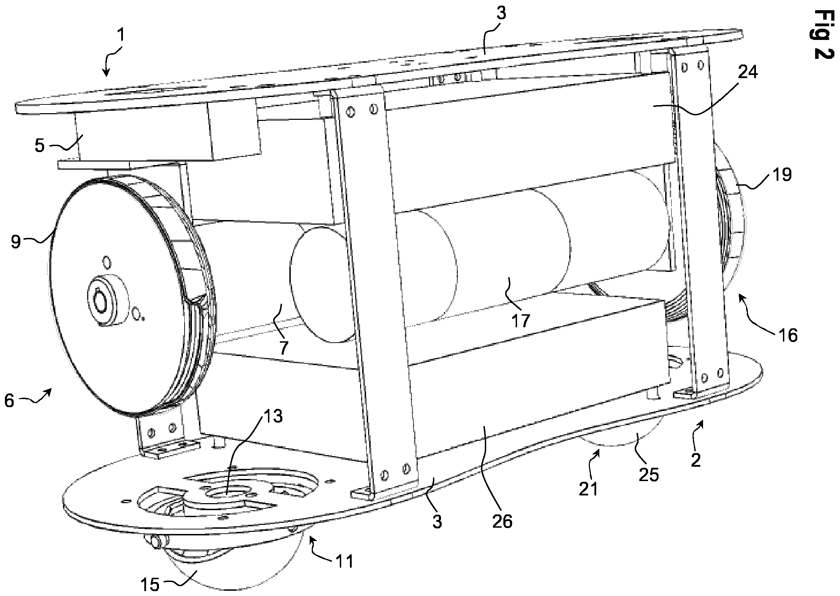

Figur 2 zeigt eine Ausführungsform einer Sensoreinrichtung mit einem ersten Positionssensor 1 und einem zweiten Positionssensor 2, die jeweils auf der in Figur 1 dargestellten Ausführungsform eines Positionssensors basieren. Dementsprechend weist der zweite Positionssensor 2 ebenfalls eine Spuleinrichtung 16 mit einer Spule 19 und einem Antrieb 17 auf, welche über eine hier nicht sichtbare Kraftmesseinrichtung mit der gemeinsamen Halterung 3 verbunden sind. Zudem weist der zweite Positionssensor 2 eine Führungseinrichtung 21 auf, von der lediglich die kardanisch gelagerte Auslassvorrichtung 25 sichtbar ist.

Die Sensoranordnung umfasst weiterhin ein Netzteil 24 und eine Elektronik 26, die zur Ansteuerung der Spuleinrichtungen 6, 16 und zur Auswertung der jeweiligen Messdaten eingerichtet ist.

Über die Sensoranordnung können unabhängig voneinander zwei jeweilige Seile gesteuert werden beziehungsweise deren Abspullänge und deren Austrittswinkel ermittelt werden, um die jeweilige Lage eines vorbestimmten Punktes auf dem zugehörigen Seil zu ermitteln.

Beispielsweise sind an den hier nicht dargestellten Seilen jeweils Schlaufen oder Schlingen vorgesehen, über die der Arm eines Patienten gehalten werden kann. Wie zuvor für die Ausführungsform in Figur 1 beschrieben, kann jeweils ein vorbestimmter Punkt, nämlich der Befestigungspunkt der Schlinge oder Schlaufe, für die beiden Positionssensoren ermittelt werden. Aus diesen beiden Punkten lässt sich ein Vektor im Raum berechnen, welcher beispielsweise die Lage eines Unterarms des Patienten im Raum widerspiegelt. Beispielsweise sind die Schlaufen oder Schlingen an den Seilen am Handgelenk und am Ellbogen des Patienten befestigt. Die Informationen über die räumliche Positionierung des Unterarms des Patienten werden beispielsweise dazu verwendet, eine Visualisierung des Arms auf einem Bildschirm darzustellen und damit eine künstliche Realität zu steuern und dort Objekte zu manipulieren. Hierzu ist die Sensoranordnung beispielsweise mit einem Computer mit entsprechender Software verbunden. Zusätzlich oder alternativ zu den dargestellten Elektromotoren 7, 17 kann der Antrieb auch eine Aufspulfeder aufweisen, welche eine definierte Kraft auf die Spulen 9, 19 und damit die Seile bewirkt.

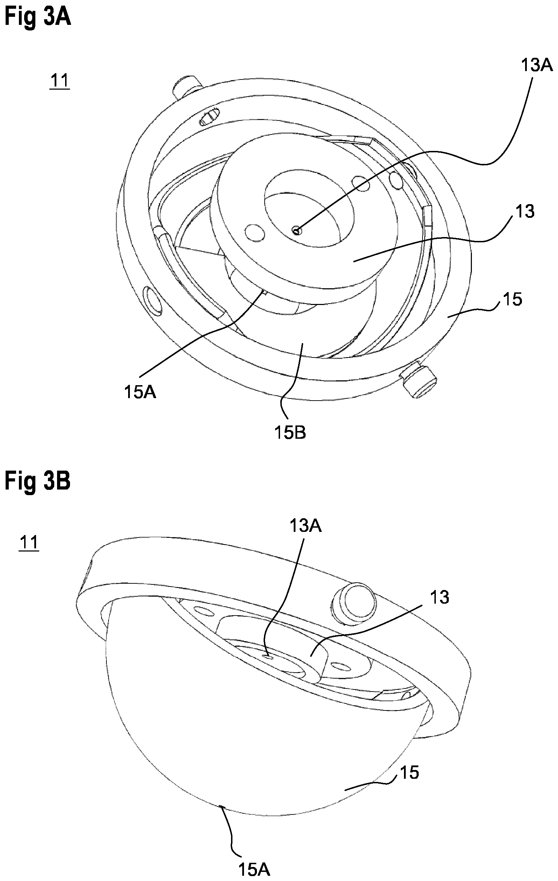

Figur 3 zeigt verschiedene perspektivische Ansichten einer Ausführungsform einer Führungseinrichtung 11, wobei in Figur 3A eine Ansicht von schräg oben und in Figur 3B eine Ansicht von schräg unten dargestellt ist. Die Führungseinrichtung 11 umfasst hierbei die Zentriereinrichtung 13 mit einem zentral angeordneten Loch 13A zur Durchführung des Seils. Ferner ist die kardanisch gelagerte Auslassvorrichtung 15 dargestellt, welche entsprechend der kardanischen Lagerung entlang von zwei Achsen schwenkbar ist. Die Zentriereinrichtung 13 bleibt auch bei einer Schwenkung der Auslassvorrichtung ortsfest. Die Auslassvorrichtung 15 weist ebenfalls ein Loch 15A zur Durchführung des Seils auf, wobei durch entsprechende Krafteinwirkung auf das Seil die Position des Lochs 15A verändert wird und sich somit die Winkellage der Auslassvorrichtung 15 verändert. Die genaue Winkellage lässt sich mit einem im Inneren der Auslassvorrichtung 15 vorgesehenen Lagesensors 15B bestimmen. Beispielsweise ist dieser Lagesensor 15B mit einem oder mehreren Beschleunigungssensoren gebildet, welche die Beschleunigung in unterschiedlichen Orientierungen bestimmen, um daraus eine Winkellage zu ermitteln. Ein solcher Lagesensor kann auch als gyroskopischer Sensor bezeichnet werden. In Figur 3B ist insbesondere die halbkugelförmige Ausgestaltung der Auslassvorrichtung 15 mit dem Ausgangsloch 15A zu erkennen.

Die in Figur 2 dargestellte Sensoranordnung lässt sich beispielsweise für ein Therapiegerät oder ein Rehabilitationsgerät verwenden, mit dem es Patienten mit verminderter Funktionsfähigkeit der oberen Extremitäten erleichtert wird, Bewegungen durchzuführen beziehungsweise wieder zu erlernen. Dies wird insbesondere durch die Entlastung des Arms des Patienten von der Schwerkraft, die ganz oder teilweise erfolgen kann, erreicht. Zudem kann durch die integrierte Positionsbestimmung der zuvor beschriebenen Schlaufen beziehungsweise Schlingen ein Bewegungsverhalten des Patienten beziehungsweise des Arms des Patienten ermittelt und gegebenenfalls aufgezeichnet werden. Die Bewegungsdaten und Positionsdaten des Arms können beispielsweise zur Motivation des Patienten grafisch visualisiert werden, um in beispielsweise im Rahmen einer virtuellen Realität zu bestimmten Bewegungen zu animieren.

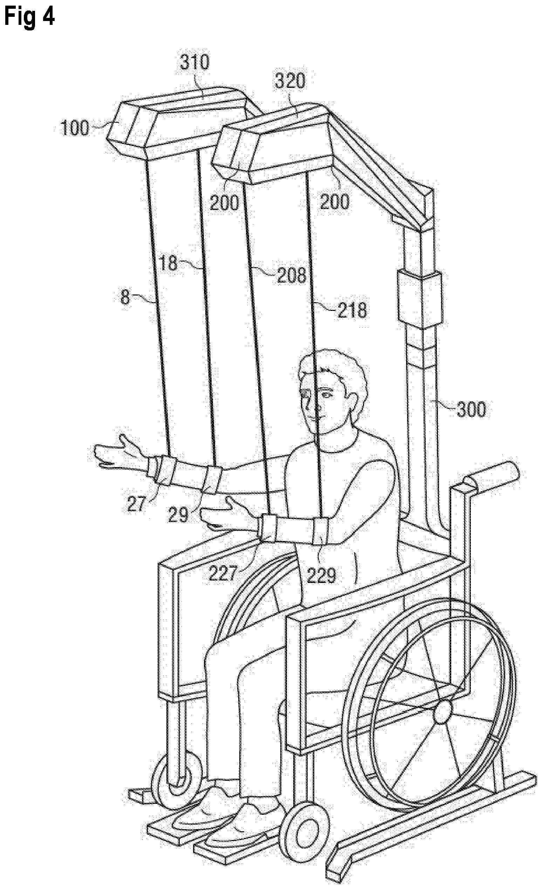

Während die in Figur 2 dargestellte Sensoranordnung zur Therapierung eines einzelnen Arms verwendet werden kann, kann bei entsprechender Kombination von zwei solchen Sensoranordnungen auch ein beidarmiger Betrieb ermöglicht werden. Figur 4 zeigt eine beispielhafte Ausführungsform eines Therapiegeräts, bei dem ein erstes und ein zweites Therapiegerät 100, 200 für den rechten beziehungsweise linken Arm eines Patienten eingesetzt wird. Die Sensoranordnungen 100, 200 sind beispielsweise an jeweiligen Halterungen 310, 320 eines Gestells 300 befestigt, welches in der dargestellten Ausführungsform an einem Rollstuhl befestigt ist, in dem der Patient sitzt. Von den Sensoranordnungen 100, 200 laufen die entsprechenden Seile 8, 18 beziehungsweise 208, 218 zum rechten beziehungsweise linken Arm des Patienten. Insbesondere ist der rechte Arm an Schlingen 27, 29 aufgenommen, während der rechte Arm in Schlingen 227, 229 aufgenommen ist.

Anstelle des Rollstuhls kann ein Gestell 300 auch an einem Krankenhausbett oder auch einem herkömmlichen Stuhl befestigt werden. Durch die Verwendung der Seile 8, 18, 208, 218 ist es während der Therapie möglich, den gesamten Raum vor dem Patienten zur Therapie zu nutzen, ohne durch das Gerät selbst dabei eingeschränkt zu werden. Insbesondere sind alle notwendigen Sensoren, die zur Bestimmung der Position des Armes notwendig sind, in den jeweiligen Sensoranordnungen 100, 200 integriert, beispielsweise im Gegensatz zu herkömmlichen Systemen, bei denen zur Positionsbestimmung eine Kamera eingesetzt wird.

Die Auswertung der Kraft des Patientenarmes ermöglicht eine jeweilige Regelung der Gewichtsentlastung, die insbesondere für den rechten und linken Arm unterschiedlich gestaltet sein kann.

Die Auswertung der Messgrößen kann innerhalb der jeweiligen Sensoranordnungen erfolgen, aber auch in einer mit den Sensoranordnungen 100, 200 verbundenen Rechnereinheit wie beispielsweise einem Computer oder einem Notebook.

## Patentansprüche

1. Rehabilitationsgerät mit zumindest einem Positionssensor (1, 2), welcher
- eine Halterung (3);
- eine fest mit der Halterung (3) verbundene Kraftmesseinrichtung (5);
- eine fest mit der Kraftmesseinrichtung (5) verbundene Spuleinrichtung (6, 16) mit einem Antrieb (7), der einen Elektromotor (7) aufweist, die Spuleinrichtung (6, 16) eingerichtet zum Abspulen und Aufspulen eines Seils (8) und zur Ermittlung einer Abspullänge des abgespulten Seils (8) und
- eine fest mit der Halterung (3) verbundene Führungseinrichtung (11) umfasst, die eine Zentriereinrichtung (13) zur definierten, insbesondere richtungsfesten Durchführung des Seils (8), eine kardanisch gelagerte Auslassvorrichtung (15) zur richtungsvariablen Durchführung des Seils (8) und einen Lagesensor zur Ermittlung einer Winkellage der Auslassvorrichtung (15) aufweist;
wobei der Positionssensor (1, 2) eingerichtet ist, die Lage eines vorbestimmten Punktes des Seils (8) relativ zur Halterung (3) auf der Basis der ermittelten Winkellage und der Abspullänge zu bestimmen und wobei der Elektromotor (7) eingerichtet ist, mit einer einstellbaren Zugkraft auf das Seil (8) einzuwirken, die auf der Basis einer Kraft eingestellt wird, welche über das Seil auf die Halterung (3) wirkt und welche von der Kraftmesseinrichtung (5) ermittelt wird.

2. Rehabilitationsgerät nach Anspruch 1, bei dem die Spuleinrichtung (6, 16) eingerichtet ist, das Seil einlagig nebeneinander oder einlagig übereinander aufzuwickeln.

3. Rehabilitationsgerät nach Anspruch 1 oder 2, bei dem der Antrieb eine Aufspulfeder aufweist.

4. Rehabilitationsgerät nach einem der Ansprüche 1 bis 3, bei dem die Abspullänge über einen mit dem Elektromotor (7) verbundenen Motorencoder bestimmt wird.

5. Rehabilitationsgerät nach einem der Ansprüche 1 bis 4, bei dem der Lagesensor wenigstens einen Beschleunigungssensor umfasst.

6. Rehabilitationsgerät nach einem der Ansprüche 1 bis 5, bei dem die Spuleinrichtung (6, 16) eingerichtet ist zum Abspulen und Aufspulen des Seils (8) auf der Basis einer Änderung der durch die Kraftmesseinrichtung (5) ermittelten Kraft.

7. Rehabilitationsgerät nach einem der Anspruche 1 bis 6, wobei ein erster Positionssensor (1) und ein zweiter Positionssensor (2) vorgesehen sind, welche in einer Sensoranordnung in einer gemeinsamen Halterung (3) angeordnet sind.

8. Rehabilitationsgerät nach Anspruch 7, bei dem die Halterung des ersten Positionssensors (1) gleichzeitig die Halterung des zweiten Positionssensors (2) ist.

9. Rehabilitationsgerät nach Anspruch 7 oder 8, das eingerichtet ist, die Lage eines Objekts relativ zur gemeinsamen Halterung (3) auf der Basis der Lage des vorbestimmten Punktes des Seils (8) des ersten Positionssensors (1) und der Lage des vorbestimmten Punktes des Seils (18) des zweiten Positionssensors (2) zu bestimmen.

10. Rehabilitationsgerät nach Anspruch 9, das eingerichtet ist, die Lage des Objekts grafisch auf einer Anzeigevorrichtung zu visualisieren.

11. Rehabilitationsgerät nach Anspruch 9 oder 10, das eingerichtet ist, die Lage des Objekts als Ortsvektor des Objekts im Raum zu berechnen.

12. Rehabilitationsgerät nach einem der Ansprüche 7 bis 11, bei dem an dem vorbestimmten Punkt des Seils (8) des ersten Positionssensors (1) und dem vorbestimmten Punkt des Seils (18) des zweiten Positionssensors (2) jeweils eine Schlinge oder Schlaufe (27, 29, 227, 229) zur Aufnahme einer menschlichen oder tierischen Extremität, insbesondere eines Arms, vorgesehen ist.

13. Rehabilitationsgerät nach Anspruch 12, wobei zumindest zwei Sensoranordnungen vorgesehen sind.

14. Verwendung eines Positionssensors für ein Rehabilitationsgerät, wobei der Positionssensor
- eine Halterung (3);
- eine fest mit der Halterung (3) verbundene Kraftmesseinrichtung (5);
- eine fest mit der Kraftmesseinrichtung (5) verbundene Spuleinrichtung (6, 16) mit einem Antrieb (7), der einen Elektromotor (7) aufweist, die Spuleinrichtung (6, 16) eingerichtet zum Abspulen und Aufspulen eines Seils (8) und zur Ermittlung einer Abspullänge des abgespulten Seils (8) und
- eine fest mit der Halterung (3) verbundene Führungseinrichtung (11) umfasst, die eine Zentriereinrichtung (13) zur definierten, insbesondere richtungsfesten, Durchführung des Seils (8), eine kardanisch gelagerte Auslassvorrichtung (15) zur richtungsvariablen Durchführung des Seils (8) und einen Lagesensor zur Ermittlung einer Winkellage der Auslassvorrichtung (15) aufweist;
wobei der Positionssensor (1, 2) eingerichtet ist, die Lage eines vorbestimmten Punktes des Seils (8) relativ zur Halterung (3) auf der Basis der ermittelten Winkellage und der Abspullänge zu bestimmen und wobei der Elektromotor (7) eingerichtet ist, mit einer einstellbaren Zugkraft auf das Seil (8) einzuwirken, die auf der Basis einer Kraft eingestellt wird, welche über das Seil auf die Halterung (3) wirkt und welche von der Kraftmesseinrichtung (5) ermittelt wird.

## Claims

1. A rehabilitation
device with at least one position sensor (1, 2), which has
- a mount (3),
- a force measuring device (5) fixedly connected with the mount (3);
- a winding device (6, 16) fixedly connected with the mount (3) with a drive (7) having an electric motor (7), the winding device (6, 16) being set up to unwind and wind a rope (8) and to determine an unwinding length of the unwound rope (8), and
- a guiding device (11) fixedly connected with the mount (3), which has a centering device (13) for guiding through the rope (8) in a defined, in particular directionally fixed manner, a gimbal-mounted outlet device (15) for guiding through the rope (8) in a directionally variable manner, and a location sensor for determining an angular position of the outlet device (15);
wherein the position sensor (1, 2) is set up to determine the position of a predetermined point of the rope (8) relative to the mount (3) based on the ascertained angular position and unwinding length, and wherein the electric motor (7) is set up to act on the rope (8) with an adjustable tensile force that is set based upon a force that acts via the rope on the mount (3) and determined by the force measuring device (5).

2. The rehabilitation device according to claim 1, in which the winding device (6, 16) is set up to wind the rope single-layered side by side or single-layer one on top of the other.

3. The rehabilitation device according to claim 1 or 2, in which the drive has a winding spring.

4. The rehabilitation device according to one of claims 1 to 3, in which the unwinding length is determined by means of motor encoder connected with the electric motor (7).

5. The rehabilitation device according to one of claims 1 to 4, in which the location sensor has at least one acceleration sensor.

6. The rehabilitation device according to one of claims 1 to 5, in which the winding device (6, 16) is set up to unwind and wind the rope (8) based on a change in the force ascertained by the force measuring device (5).

7. The rehabilitation device according to one of claims 1 to 6, wherein a first position sensor (1) and a second position sensor (2) are provided, which are arranged in a sensor arrangement in a shared mount (3).

8. The rehabilitation device according to claim 7, in which the mount for the first position sensor (1) is simultaneously the mount for the second position sensor (2).

9. The rehabilitation device according to claim 7 or 8, which is set up to determine the position of an object relative to the shared mount (3) based on the position of the predetermined point of the rope (8) of the first position sensor (1) and the position of the predetermined point of the rope (18) of the second position sensor (2).

10. The rehabilitation device according to claim 9, which is set up to visualize the position of the object graphically on a display device.

11. The rehabilitation device according to claim 9 or 10, which is set up to calculate the position of the object as a position vector of the object in space.

12. The rehabilitation device according to one of claims 7 to 11, in which a respective sling or loop (27, 29, 227, 229) is provided on the predetermined point of the rope (8) of the first position sensor (1) and on the predetermined point of the rope (18) of the second position sensor (2) for accommodating a human or animal extremity, in particular an arm.

13. The rehabilitation device according to claim 12, wherein at least two sensor arrangements are provided.

14. Use of a position sensor for a rehabilitation device, wherein the position sensor has
- a mount (3),
- a force measuring device (5) fixedly connected with the mount (3);
- a winding device (6, 16) fixedly connected with the mount (3) with a drive (7) having an electric motor (7), the winding device (6, 16) set up to unwind and wind a rope (8) and to determine a n unwinding length of the unwound rope (8), and
- a guiding device (11) fixedly connected with the mount (3), which has a centering device (13) for guiding through the rope (8) in a defined, in particular directionally fixed manner, a gimbal-mounted outlet device (15) for guiding through the rope (8) in a directionally variable manner, and a location sensor for determining an angular position of the outlet device (15);
wherein the position sensor (1, 2) is set up to determine the position of a predetermined point of the rope (8) relative to the mount (3) based on the ascertained angular position and unwinding length, and wherein the electric motor (7) is set up to act on the rope (8) with an adjustable tensile force that is set based upon a force that acts via the rope on the mount (3) and determined by the force measuring device (5).

## Revendications

1. Appareil de rééducation avec au moins un capteur de position (1, 2) comprenant :
- un support (3) ;
- un dispositif de mesure de force (5) relié fixement au support (3) ;
- un dispositif d'enroulage (6, 16) relié fixement au dispositif de mesure de force (5) avec un entraînement (7) comportant un moteur électrique (7), le dispositif d'enroulage (6, 16) étant conçu pour dérouler et enrouler un câble (8) et pour déterminer une longueur de déroulage du câble déroulé (8), et
- un dispositif de guidage (11) relié fixement au support (3), lequel comporte un dispositif de centrage (13) pour le passage défini du câble (8), en particulier fixe quant à la direction, un dispositif de déchargement (15) monté de façon cardanique pour le passage du câble (8) de façon variable quant à la direction, et un capteur de position chargé de détecter une position angulaire du dispositif de déchargement (15) ;
dans lequel le capteur de position (1, 2) est conçu pour détecter la position d'un point prédéfini du câble (8) par rapport au support (3) sur la base de la position angulaire détectée et de la longueur de déroulage, et dans lequel le moteur électrique (7) est conçu pour agir sur le câble (8) avec une force de traction réglable, laquelle est réglée sur la base d'une force agissant sur le support (3) par le biais du câble et déterminée par le dispositif de mesure de force (5).

2. Appareil de rééducation selon la revendication 1, dans lequel le dispositif d'enroulage (6, 16) est conçu pour enrouler le câble en une seule couche de façon juxtaposée ou en une seule couche de façon superposée.

3. Appareil de rééducation selon la revendication 1 ou 2, dans lequel l'entraînement comporte un ressort d'enroulage.

4. Appareil de rééducation selon l'une des revendications 1 à 3, dans lequel la longueur de déroulage est déterminée par le biais d'un codeur de moteur relié au moteur électrique (7).

5. Appareil de rééducation selon l'une des revendications 1 à 4, dans lequel le capteur de position comprend au moins un capteur d'accélération.

6. Appareil de rééducation selon l'une des revendications 1 à 5, dans lequel le dispositif d'enroulage (6, 16) est conçu pour l'enroulage et le déroulage du câble (8) sur la base d'une modification de la force déterminée par le dispositif de mesure de force (5).

7. Appareil de rééducation selon l'une des revendications 1 à 6, dans lequel il est prévu un premier capteur de position (1) et un deuxième capteur de position (2), lesquels sont disposés dans un ensemble de capteurs dans un support commun (3).

8. Appareil de rééducation selon la revendication 7, dans lequel le support du premier capteur de position (1) est également le support du deuxième capteur de position (2).

9. Appareil de rééducation selon la revendication 7 ou 8, lequel est conçu pour déterminer la position d'un objet par rapport au support commun (3) sur la base de la position du point prédéfini du câble (8) du premier capteur de position (1) et de la position du point prédéfini du câble (18) du deuxième capteur de position (2).

10. Appareil de rééducation selon la revendication 9, lequel est conçu pour visualiser la position de l'objet graphiquement sur un dispositif d'affichage.

11. Appareil de rééducation selon la revendication 9 ou 10, lequel est conçu pour calculer la position de l'objet en tant que vecteur de position de l'objet dans l'espace.

12. Appareil de rééducation selon l'une des revendications 7 à 11, dans lequel une boucle ou un noeud coulant (27, 29, 227, 229) destiné à recevoir une extrémité humaine ou animale, en particulier d'un bras, est prévu(e) respectivement au point prédéfini du câble (8) du premier capteur de position (1) et au point prédéfini du câble (18) du deuxième capteur de position (2).

13. Appareil de rééducation selon la revendication 12, dans lequel il est prévu au moins deux ensembles de capteurs.

14. Utilisation d'un capteur de position pour un appareil de rééducation, dans laquelle le capteur de position comprend :
- un support (3) ;
- un dispositif de mesure de force (5) relié fixement au support (3) ;
- un dispositif d'enroulage (6, 16) relié fixement au dispositif de mesure de force (5) avec un entraînement (7) comportant un moteur électrique (7), le dispositif d'enroulage (6, 16) étant conçu pour dérouler et enrouler un câble (8) et pour déterminer une longueur de déroulage du câble déroulé (8), et
- un dispositif de guidage (11) relié fixement au support (3), lequel comporte un dispositif de centrage (13) pour le passage défini du câble (8), en particulier fixe quant à la direction, un dispositif de déchargement (15) monté de façon cardanique pour le passage du câble (8) de façon variable quant à la direction, et un capteur de position chargé de détecter une position angulaire du dispositif de déchargement (15) ;
dans laquelle le capteur de position (1, 2) est conçu pour détecter la position d'un point prédéfini du câble (8) par rapport au support (3) sur la base de la position angulaire détectée et de la longueur de déroulage, et dans lequel le moteur électrique (7) est conçu pour agir sur le câble (8) avec une force de traction réglable, laquelle est réglée sur la base d'une force agissant sur le support (3) par le biais du câble et déterminée par le dispositif de mesure de force (5).
